# EUROPEAN PATENT APPLICATION

(11) **EP 1 891 976 A1**
(43) Date of publication of application: **27.02.2008**
(21) Application number: 06119420.5
(22) Date of filing: 23.08.2006
(51) Int. Cl.: A61K 48/00, C12N 15/864, C07K 14/415

(54) **Use of light sensitive genes**

(71) Applicant: Novartis Forschungsstiftung, Zweigniederlassung, 4058 Basel (CH)
(72) Inventor: The designation of the inventor has not yet been filed
(74) Representative: de Weerd, Petrus G.W.

(57) **Abstract**

The invention relates to the use of a light-gated ion channel for the manufacture of a medicament for the treatment of blindness and a method for expressing said cell specific fashion, e.g. in ON-bipolar cells, ON-ganglion cells, or All amacrine cells.

## Description

### Field of the Invention

The present invention relates to a method of treating blindness. The present invention also relates to constructs for use in treating blindness, as well as their use in the manufacture of a medicament for treating blindness.

### Background of the Invention

Blindness is a major health problem that disables millions of people worldwide. The most common cause of blindness is the disfunction of the retina. The three most common forms of retinal blindness are retinitis pigmentosa (RP), macular deneneration (MD) and glaucoma (G). In RP and MD the primary problem is the degeneration of photoreceptors and the consequent loss of photosensitivity. There is thus a need to be able to obviate the problems associated with such degeneration of photoreceptors.

One approach has been to develop a retinal prosthesis, a "seeing eye" chip with as many as 1,000 tiny electrodes to be implanted in the eye. This would have the potential to help people who have lost their sight to regain enough vision to function independently, but the numbers of electrodes is simply insufficient to provide a high degree or level of sight to be obtained. Moreover, there are problems associated with inserting foreign bodies into the eye.

Recently a number of genes has been isolated and/or manipulated that when expressed can make cells light sensitive. In some cases additional non-genetic factors are also needed to make cells light sensitive.

One proposal by Eli in 2001 was to use the chlorophyll - containing proteins in spinach to treat vision loss. These proteins give off a small electrical voltage after capturing the energy of incoming photons of light. Although, the research has shown that photosystem I reaction centres can be incorporated into a liposome and are shown to be functional, in that it produces the experimental equivalent of a voltage when light is shone on it, hitherto this has not been shown to work in a retinal cell.

Other work by neurobiologist Richard Kramer at UC Berkeley has looked at re-engineering a potassium channel to be responsive to light rather than voltage, in order to allow insertion of a light activated switch into brain cells normally insensitive to light. However, the channel has to be mutated so that it always stays open and a chemical "plug", attached to the channel, which is sensitive to light such that when lit with long-wavelength UV light, the plug is released from the channel, letting potassium out of the channel. Light of a longer wavelength causes the plug to insert back into the channel and stop release of potassium. It will be appreciated however, that such a system is extremely complex and problems are likely to arise if the channel is delivered to the wrong type of retinal cells.

Bi et al., (Neuron, 50, 2006, p23-33) discloses the use of microbial-type rhodopsin to restore visual responses in mice with photoreceptor degeneration. However, the expression of the rhodopsin gene is likely to have occurred in a variety of types of cell in the eye which is potentially undesirable and/or problematic. It also appears that the threshold light intensity required for producing responses is much higher than for normal rod and cone photoreceptors, but there is no teaching of how this may be addressed in, for example, low light environments.

It is amongst the objects of the present invention to obviate and/or mitigate at least one of the aforementioned disadvantages.

It is also an object of the present invention to provide a system suitable for use in treating blindness in a subject.

### Summary of the Invention

In a first aspect there is provided use of a light-gated ion channel gene or active fragment thereof for the manufacture of a medicament for use in treating or ameliorating blindness.

It is to be understood that the medicament is generally used therapeutically, but it may be used in a prophylactic sense, when a subject has been identified as being likely to suffer from blindness, but actual vision loss has not yet occurred or has only minimally occurred.

By blindness is meant total or partial loss of vision. Typically the medicament may be used to treat blindness associated with macular degeneration, glaucoma and/or retinitis pigmentosa. However, it is to be appreciated that any disease or condition which leads to degeneration or non-functioning of photoreceptors in the eye may be treated using the medicament.

An active fragment of the light- gated ion channel is a fragment which when expressed generates a polypeptide which is still capable of functioning as a light capturing molecule which causes a subsequent flow of ions into or out of the cell in which the channel is located and a consequent change in voltage.

It will be appreciated that the present invention also extends to methods of treating prophylactically or therapeutically blindness by administering to a patient suffering or predisposed to developing blindness, a DNA construct comprising a light-gated ion channel gene sequence or active fragment thereof, which gene sequence or fragment thereof is capable of expressing one or more copies of the light- gated ion channel protein in a retinal cell, whereby expression of said one or more copies of the light- gated ion channel protein render the cell photosensitive so as to enable treatment or amelioration of blindness.

Typically, the light-gated ion channel gene sequence or fragment thereof may be administered to a subject in the form of a recombinant molecule comprising said light-gated ion channel gene sequence or active fragment under appropriate transcriptional/translational controls to allow expression of said light- gated ion channel protein when administered to retinal cells of a subject. It will be appreciated that the light- gated ion channel sequence or fragment may be under control of a suitable promoter, such as a constitutive and/or controllable promoter.

The present invention also therefore provides a recombinant molecule comprising an light- gated ion channel gene sequence or active fragment thereof for use in therapy. The recombinant molecule may be in the form of a plasmid, phagemid or viral vector. Furthermore, recombinantly expressed, or chemically synthesised light-gated ion channel protein, or functionally important fragments thereof, may be produced and applied to the eye via a suitable ointment or other pharmaceutical vehicle, as a treatment or prophylactic measure for treating said aforementioned diseases.

Many different viral and non-viral vectors and methods of their delivery, for use in gene therapy, are known, such as adenovirus vectors, adeno-associated virus vectors, retrovirus vectors, lentiviral vectors, herpes virus vectors, liposomes, naked DNA administration and the like. A detailed review of possible techniques for transforming genes into desired cells of the eye is taught by Wright (Br J Ophthalmol, 1997; 81: 620-622) which is incorporated herein by reference. Moreover, it may also be possible to use encapsulated cell technology as developed by Neurotech, for example.

Desirably the light-gated ion channel gene is a rhodopsin gene, such as a rhodopsin from a microorganism, such as a unicellular alga, typically from the species Chlamydononas, especially Chlamydomonas reinhardtii. A preferred rhodopsin is Channelrhodopsin - 2 (ChR2) which is a light gated cation channel from C. reinhardtii, see for example, Boyden et al 2005 (Nature Neuroscience, 8, 9; 1263-1268), incorporated herein by reference.

Preferably the cells to which the medicament or vector are to be administered, and in which the gene is to be expressed are ON-bipolar cells, ON-ganglion, or AII amacrine cells. Moreover, the photoreceptor cells themselves which have lost photosensitivity, but which are not "dead" could be used to express the light- gated ion channel gene, though the polarity of the response would be the opposite than under normal conditions. Moreover expression of the light-gated ion channel gene in photoreceptors may serve to prevent or show down degeneration.

It is understood that it is preferable that expression of said light- gated ion channel gene is controlled by way of a cell specific promoter. Thus a cell specific promoter may be used to ensure that the light- gated ion channel gene is only expressed in a specific cell type. For example, the mGluR6 promoter (Ueda et al, J Neurosci. 1997 May 1;17(9):3014-23)may be employed to control expression in ON-bipolar cells.

Once expressed in an appropriate retinal cell, the light- gated ion channel protein inserts within the plasma membrane of the cell, rendering the cell photosensitive and able to cause ion transport, cation or anion, in response to light. Nevertheless, although it is known that the retina is sensitive to very wide ranges of light intensities due to the adaptive nature of photoreceptors, light-gated ion channels may not be able to adapt and may therefore respond only to a narrow range of light intensities. If this is the case, such a limitation may be mitigated by use of image intensifiers and/or image converters known in the art. For example, a patient who has been treated by the above described method, may wear, image intensifiers/enhancers mounted, for example, on spectacles or the like.

By way of an example, an image intensifying device, such as those provided by Telesensory (http://www.telesensory.com), may be combined with a retinal scanning device (RSD) as developed by Microvision (http://www.microvision.com/milprod.html), to provide a head-worn apparatus capable of delivering a bright, intensified image directly to the retina of a patient with impaired vision (http://www.telesensory.com/home8.html). Briefly, a RSD projects images onto the retina such that an individual can view a large, full-motion image without the need for additional screens or monitors. Thus, by projecting an intensified image directly to the retina of an individual with impaired vision, it may be possible to improve vision in those considered to be blind.

In case of expressing the light-gated ion channel in retinal bipolar or ganglion cells some aspects of the network processing capabilities of the retina are lost. For example horizontal cell mediated lateral inhibition is lost if light activates bipolar or ganglion cells. In these cases a retina like processor (D. Balya and B. Roska: "Retina model with real time implementation", International Symposium on Circuits and Systems ISCAS 2005, Kobe, Japan, May, pp. 5222-5225., also see http://www.anafocus.com/ and http://www.eutecus.com/ ) can be combined with the Microvision system.

If the light- gated ion channel is expressed in photoreceptors as mentioned before the polarity of light response in photoreceptors inverses. That can be corrected with inverting the polarity of the projected image: dark pixels becoming light and light pixels becoming dark.

### Detailed Description of the Invention

The present invention will now be described by way of example and with reference to the following figure:

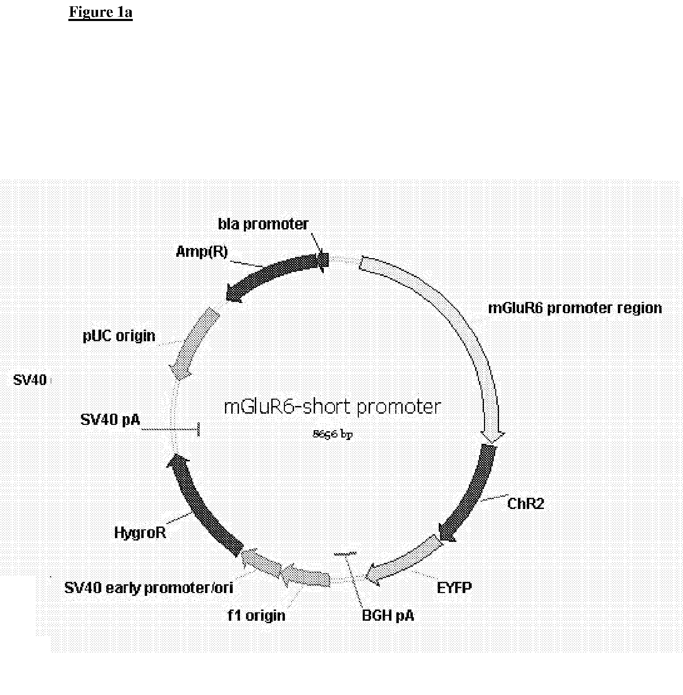

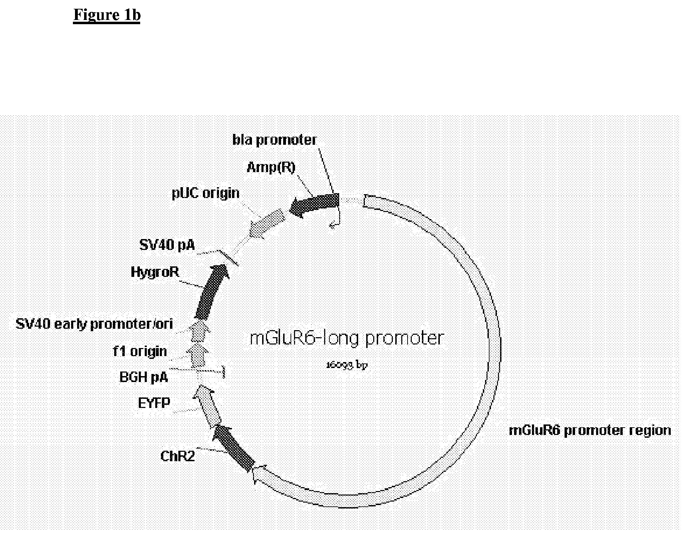

Figures 1a & b show schematic maps of constructs for use in the present invention.

### Plasmid preparation

The metabotropic glutamate receptor 6 (mGluR6) gene is specifically expressed in certain types of bipolar cells, called ON bipolar cells, within the inner retina. These cells mediate responsiveness to a light signal that is relayed by the photoreceptor cells. The regulatory sequences of the mGluR6 gene are responsible for its cell specific expression. These regulatory sequences or functional fragments or derivatives thereof can be used to direct the production of another gene to make the ON bipolar cells sensitive to light in the absence of functioning photoreceptor cells. Promoter analysis can be used to identify promoter functional fragments and derivatives (McGowen at al. Mol. Vision 1998, 4:2; Bookstein et al. PNAS 1990, 87 (19); 7762-66)

The expression plasmids consist of a mGluR6 promoter sequence (Ueda et al) linked to the channelrhodopsin-2 protein-coding sequence, which in turn is fused to a sequence encoding a fluorescent protein. This fluorescent "tag" enables the visualization of which cells are producing the channelrhodopsin-2 protein. All of these sequences are combined with additional DNA elements that comprise a vector for transport to and protein production within mammalian cells. Examples of suitable constructs are shown in Figures 1a and 1b.

### Plasmid delivery

The plasmids can be delivered to the retinas of mice by a technique called electroporation. First, a pure preparation of the plasmid can be injected into the subretinal space of the mouse eye. Then an electrical current can be applied to the eye to cause the DNA to enter the retina. The DNA remains there and can be expressed for several weeks.

Another method of delivery of the channelrhodopsin-2 protein into the bipolar cells is to use a viral-based mechanism. Recombinant adeno-associated viruses can be produced that encode the channelrhodopsin-2 protein linked to the mGluR6 promoter region. The viruses can be injected into the intravitreal space of the mouse eye. The virus particles would infect cells of the retina, and gene expression would be tested several weeks later.

The plasmids and recombinant adeno-associated viruses can be delivered to the retinas of mice that are blind as a result of a genetic mutation that causes retinal degeneration (Bowes et al., Nature, 1990; 347;667-680). The photoreceptor cells of these mice are lost within a few weeks after birth. These mice are a frequently used experimental model system for studying retinal degenerative diseases that affect humans. By introducing channelrhodopsin-2 into the bipolar cells of these animals, the aim is to restore light sensitivity to the otherwise photo-insensitive retinas. Therefore these studies may serve as the basis for gene therapy approaches for human cases of retinal disease.

Once a construct is delivered to ON bipolar cells, activity of ganglion cells may be recorded, the output cells of the retina which provide information about the visual scene to the rest of the brain. An array of electrodes or a single electrode can be used to record activity from ganglion cells. In normal mice, ganglion cells produce electric "spikes" when stimulated by light. The retina, can be prepared to express the light sensitive channelrhodopsin-2 channel in ON bipolar cells, from the eye of the blind mice and stimulated with light. During light stimulation the spiking activity from ganglion cells can be recorded. If spiking activity evoked by light can be measured, it can be concluded that the blind mice can "see".

## Claims

1. Use of a light- gated ion channel gene or active fragment thereof for the manufacture of a medicament for use in treating or ameliorating blindness.

2. Claim 1 wherein the light- gated ion channel gene is a rhodopsin gene.

3. Claim 2 wherein the rhodopsin gene is Channelrhodopsin - 2 (ChR2).

4. Any of the preceding claims wherein the light- gated ion channel gene is administered to and expressed in at least one of ON-bipolar cells, ON-ganglion, or AII amacrine cells.

5. Claims 4 wherein the light-gated ion channel gene is administered to and expressed in AII amacrine cells.

6. Any of the preceding claims wherein expression of said light- gated ion channel gene is controlled by way of a cell specific promoter.

7. Claim 7 wherein expression of the light gated-ion channel gene is controlled by the mGluR6 promoter or a functional fragment or derivate thereof.

8. A construct useful in the preparation of a medicament for treating blindness comprising; Channelrhodopsin - 2 (ChR2) under the control of a mGluR6 promoter.
